(19) Europäisches Patentamt
European Patent Office
Office européen des brevets

(11) Veröffentlichungsnummer: **0 315 009**
**A2**

(12) # EUROPÄISCHE PATENTANMELDUNG

(21) Anmeldenummer: **88117667.1**

(22) Anmeldetag: **24.10.88**

(51) Int. Cl.$^4$: **C07D 311/68 , C07D 311/60 , C07D 311/64 , C07D 311/70 , C07D 311/58 , A61K 31/35**

(30) Priorität: **03.11.87 DE 3737195**

(43) Veröffentlichungstag der Anmeldung:
**10.05.89 Patentblatt 89/19**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Niewöhner, Ulrich, Dr.**
**Gartenstrasse 3**
**D-5632 Wermelskirchen(DE)**
Erfinder: **Hoever, Franz-Peter, Dr.**
**Kunstfelder Strasse 25**
**D-5000 Koeln 80(DE)**
Erfinder: **Lieb, Folker, Dr.**
**Alfred-Kubin-Strasse 1**
**D-5090 Leverkusen 1(DE)**
Erfinder: **Oediger, Herman, Dr.**
**Roggendorfstrasse 51**
**D-5000 Koeln 80(DE)**
Erfinder: **Rosentreter, Ulrich, Dr.**
**Kondorweg 23**
**D-5600 Wuppertal 1(DE)**
Erfinder: **Böshagen, Horst, Dr.**
**Wiesenstrasse 4**
**D-5657 Haan(DE)**
Erfinder: **Perzborn, Elisabeth, Dr.**
**Am Tescher Busch 13**
**D-5600 Wuppertal 11(DE)**
Erfinder: **Fiedler, Volker-Bernd**
**Lehner Muehle 46**
**D-5090 Leverkusen 3(DE)**
Erfinder: **Seuter, Friedel, Dr.**
**Moospfad 16**
**D-5600 Wuppertal 1(DE)**

(54) **Chromanderivate, Verfahren zu ihrer Herstellung und ihre Verwendung in Arzneimitteln.**

(57) Die Erfindung betrifft neue Chromanderivate der allgemeinen Formel I

**EP 0 315 009 A2**

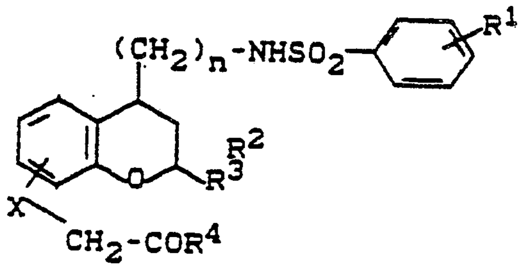

(I)

in welcher $R^1$ bis $R^4$, X und n die in der Beschreibung angegebene Bedeutung haben, ein Verfahren zu ihrer Herstellung und ihre Verwendung in Arzneimitteln, insbesondere als Thrombosemittel.

## Chromanderivate, Verfahren zu ihrer Herstellung und ihre Verwendung in Arzneimitteln

Die Erfindung betrifft neue Chromanderivate, ein Verfahren zu ihrer Herstellung und ihre Verwendung in Arzneimitteln, insbesondere als Thrombosemittel und als Arzneimittel gegen asthmatische Erkrankungen. Es ist bereits bekannt, daß bestimmte Chromanderivate interessante pharmazeutische Wirkungen zeigen. In der DE-OS 3 411 993 werden z.B. Chromanderivate beschrieben mit blutdrucksenkender Wirkung.

Gegenstand der Erfindung sind neue Chromanderivate der allgemeinen Formel (I)

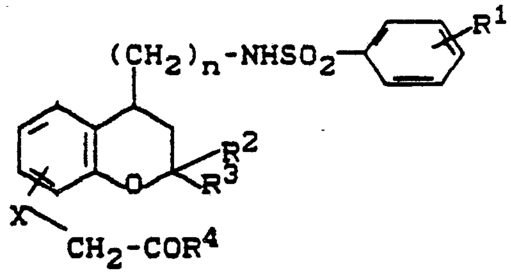

(I)

in welcher

$R^1$ für Wasserstoff, Halogen, Alkyl, Hydroxy, Alkoxy, Trifluormethyl oder Cyano steht,

$R^2$ und $R^3$ gleich oder verschieden sind und für Wasserstoff, Alkyl, Cycloalkyl oder Aryl stehen,

$R^4$ für Hydroxy, Alkoxy, Aryloxy, Aralkoxy oder für eine Gruppe der Formel $-NR^5R^6$ steht, wobei $R^5$ und $R^6$ gleich oder verschieden sind und jeweils Wasserstoff, Alkyl, Aryl oder Aralkyl bedeuten,

X eine direkte Bindung oder ein Sauerstoff- oder Schwefelatom, NH oder N-Alkyl bedeutet, und

n 0 oder 1 bedeutet,

sowie deren physiologisch verträglichen Salze.

Alkyl steht im allgemeinen für einen verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Aryl steht im allgemeinen für einen aromatischen Rest mit 6 bis etwa 12 Kohlenstoffatomen. Bevorzugte Arylreste sind Phenyl, Naphthyl und Biphenyl.

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt:

Benzyloxy, Naphthylmethoxy, Phenethoxy und Phenylpropoxy.

Halogen steht im allgemeinen für Fluor, Chlor, Brom oder Iod, bevorzugt für Fluor, Chlor oder Brom. Besonders bevorzugt steht Halogen für Fluor oder Chlor.

Bevorzugt werden Verbindungen der allgemeinen Formel (I), in welcher

$R^1$ für Wasserstoff, Fluor, Chlor, Brom, Jod, Methyl, Ethyl, Propyl, Isopropyl, Hydroxy, Methoxy, Ethoxy, Propoxy, Isopropoxy, Trifluormethyl oder Cyano steht,

$R^2$ und $R^3$ gleich oder verschieden sind und für Wasserstoff, Methyl, Ethyl, Propyl, Isopropyl, Cyclobutyl, Cyclopentyl, Cyclohexyl, Cycloheptyl oder Phenyl stehen,

$R^4$ für Hydroxy, Methoxy, Ethoxy, Phenoxy, Benzyloxy oder für eine Gruppe der Formel $-NR^5R^6$ steht, wobei $R^5$ und $R^6$ gleich oder verschieden sind und jeweils Wasserstoff, Methyl, Ethyl, Phenyl oder Benzyl bedeuten,

X für eine direkte Bindung oder ein Sauerstoff- oder Schwefelatom steht,
n für 0 oder 1 steht
und deren Salze.

Die erfindungsgemäßen Stoffe zeigen überraschenderweise eine thrombozytenaggregationshemmende Wirkung und können zur Behandlung von thromboembolischen Erkrankungen verwendet werden. Weiterhin wirken sie bronchodilatorisch und können daher auch zur Behandlung von asthmatischen Erkrankungen eingesetzt werden.

Die erfindungsgemäßen Chromanderivate können auch in Form ihrer Salze vorliegen. Im allgemeinen seien hier Salze mit anorganischen oder organischen Basen genannt.

Im Rahmen der vorliegenden Erfindung werden physiologisch unbedenkliche Salze bevorzugt. Physiologisch unbedenkliche Salze der Chromanderivate können Metall- oder Ammoniumsalze der erfindungsgemäßen Stoffe sein, welche eine freie Carboxylgruppe tragen ($R^4$ = OH). Besonders bevorzugt sind z.B. Natrium-, Kalium-, Magnesium- oder Calciumsalze, sowie Ammoniumsalze, die abgeleitet sind von Ammoniak oder organischen Aminen, wie beispielsweise Ethylamin, Diethylamin, Triethylamin, Diethanolamin, Triethanolamin, Dicyclohexylamin, Dimethylaminoethanol, Arginin oder Ethylendiamin.

Die neuen Chromanderivate können sowohl als Enantiomere, Enantiomerenpaar bzw. bei Vorliegen eines weiteren Asymetriezentrums in einem der Reste als Diastereomerenpaar vorliegen.

Beispielhaft seien folgende neue Chroman- bzw. Thiochromanderivate genannt:

4-(Phenylsulfonylaminomethyl)-chroman-7-yl-oxyessigsäure
4-(3-Fluorphenylsulfonylaminoethyl)-chroman-7-yl-oxyessigsäure
4-(4-Methylphenylsulfonylaminomethyl)-chroman-7-yl-oxyessigsäure
4-(4-Ethylphenylsulfonylaminomethyl)-chroman-7-yl-oxyessigsäure
4-(4-Fluorphenylsulfonylaminomethyl)-2,2-dimethyl-chroman-7-yl-oxyessigsäure
4-(Phenylsulfonylaminomethyl)-2,2-dimethyl-chroman-7-yl-oxyessigsäure
4-(4-Methylphenylsulfonylaminomethyl)-2,2-dimethyl-chroman-7-yl-oxyessigsäure
4-(Phenylsulfonylaminomethyl)-chroman-8-yl-essigsäure
4-(4-Fluorphenylsulfonylaminomethyl)-chroman-8-yl-essigsäure
4-(3-Fluorphenylsulfonylaminomethyl)-chroman-8-yl-essigsäure
4-(4-Chlorphenylsulfonylaminomethyl)-chroman-8-yl-essigsäure
4-(3-Chlorphenylsulfonylaminomethyl)-chroman-8-yl-essigsäure
4-(4-Methylphenylsulfonylaminomethyl)-chroman-8-yl-essigsäure
4-(4-Trifluormethylphenylsulfonylaminomethyl)-chroman-8-yl-essigsäure
4-(Phenylsulfonylaminomethyl)-2,2-dimethyl-chroman-8-yl-essigsäure
4-(4-Fluorphenylsulfonylaminomethyl)-2,2-dimethyl-chroman-8-yl-essigsäure
4-(4-Chlorphenylsulfonylaminomethyl)-2,2-dimethyl-chroman-8-yl-essigsäure
4-(4-Methylphenylsulfonylaminomethyl)-2,2-dimethyl-chroman-8-yl-essigsäure
4-(Phenylsulfonylaminomethyl)-chroman-8-yl-oxyessigsäure
4-(4-Chlorphenylsulfonylaminomethyl)-chroman-8-yl-oxyessigsäure
4-(3-Fluorphenylsulfonylaminomethyl)-chroman-8-yl-oxyessigsäure
4-(4-Methylphenylsulfonylaminomethyl)-chroman-8-yl-oxyessigsäure
4-(4-Ethylphenylsulfonylaminomethyl)-chroman-8-yl-oxyessigsäure
4-(4-Methoxyphenylsulfonylaminomethyl)-chroman-8-yl-oxyessigsäure
4-(4-Cyanophenylsulfonylaminomethyl)-chroman-8-yl-oxyessigsäure
4-(4-Trifluormethylphenylsulfonylaminomethyl)-chroman-8-yl-oxyessigsäure
4-(Phenylsulfonylaminomethyl)-2,2-dimethyl-chroman-8-yl-oxyessigsäure
4-(4-Fluorphenylsulfonylaminomethyl)-2,2-dimethyl-chroman-8-yl-oxyessigsäure
4-(4-Chlorphenylsulfonylaminomethyl)-2,2-dimethyl-chroman-8-yl-oxyessigsäure
4-(Phenylsulfonylaminomethyl)-2,2-spirocyclopentyl-chroman-8-yl-oxyessigsäure
4-(4-Fluorphenylsulfonylaminomethyl)-2,2-spirocyclopentyl-chroman-8-yl-oxyessigsäure
4-(4-Chlorphenylsulfonylaminomethyl)-2,2-spirocyclopentyl-chroman-8-yl-oxyessigsäure
4-(Phenylsulfonylaminomethyl)-2,2-dimethyl-chroman-7-yl-essigsäure
4-(3-Fluorphenylsulfonylaminomethyl)-2,2-dimethyl-chroman-7-yl-essigsäure
4-(4-Cyanophenylsulfonylaminomethyl)-2,2-dimethyl-chroman-7-yl-essigsäure
4-(4-Methoxyphenylsulfonylaminomethyl)-2,2-dimethyl-chroman-7-yl-essigsäure
4-(4-Fluorphenylsulfonylaminomethyl)-2-phenyl-chroman-7-yl-oxyessigsäure
4-(Phenylsulfonylamino)-chroman-7-yl-oxyessigsäure
4-(4-Fluorphenylsulfonylamino)-chroman-7-yl-oxyessigsäure
4-(4-Chlorphenylsulfonylamino)-chroman-7-yl-oxyessigsäure

4-(4-Trifluormethylphenylsulfonylamino)-chroman-7-yl-oxyessigsäure
4-(4-Methylphenylsulfonylamino)-chroman-7-yl-oxyessigsäure
4-(3-Fluorphenylsulfonylamino)-chroman-7-yl-oxyessigsäure
4-(Phenylsulfonylaminomethyl)-chroman-7-yl-essigsäure
4-(4-Fluorphenylsulfonylaminomethyl)-chroman-7-yl-essigsäure
4-(4-Methylphenylsulfonylaminomethyl)-chroman-7-yl-essigsäure
4-(3-Chlorphenylsulfonylaminomethyl)-chroman-7-yl-essigsäure
4-(Phenylsulfonylamino)-2,2-dimethyl-chroman-7-yl-oxyessigsäure
4-(4-Ethylphenylsulfonylamino)-2,2-dimethyl-chroman-7-yl-oxyessigsäure
4-(4-Methoxyphenylsulfonylamino)-2,2-dimethyl-chroman-7-yl-oxyessigsäure
4-(4-Cyanophenylsulfonylamino)-2,2-dimethyl-chroman-7-yl-oxyessigsäure
4-(3-Fluorphenylsulfonylamino)-2,2-dimethyl-chroman-7-yl-oxyessigsäure
4-(3-Chlorphenylsulfonylamino)-2,2-dimethyl-chroman-7-yl-oxyessigsäure
4-(3-Methylphenylsulfonylamino)-2,2-dimethyl-chroman-7-yl-oxyessigsäure
4-(Phenylsulfonylaminomethyl)-2,2-spirocyclopentyl-chroman-7-yl-oxyessigsäure
4-(4-Fluorphenylsulfonylaminoethyl)-2,2-spirocyclopentyl-chroman-7-yl-oxyessigsäure
4-(4-Methylphenylsulfonylaminomethyl)-2,2-spirocyclopentyl-chroman-7-yl-oxyessigsäure
4-(4-Fluorphenylsulfonylamino)-2,2-spirocyclohexyl-chroman-7-yl-oxyessigsäure
4-(Phenylsulfonylaminomethyl)-2,2-spirocyclohexyl-chroman-7-yl-oxyessigsäure
4-(Phenylsulfonylamino)-chroman-8-yl-oxyessigsäure
4-(4-Chlorphenylsulfonylamino)-chroman-8-yl-oxyessigsäure
4-(3-Fluorphenylsulfonylamino)-chroman-8-yl-oxyessigsäure
4-(4-Methylphenylsulfonylamino)-chroman-8-yl-oxyessigsäure
4-Phenylsulfonylamino)-2,2-dimethyl-chroman-8-yl-oxyessigsäure
4-(4-Fluorphenylsulfonylamino)-2,2-dimethyl-chroman-8-yl-oxyessigsäure
4-(4-Chlorphenylsulfonylamino)-2,2-dimethyl-chroman-8-yl-oxyessigsäure
4-(4-Methylphenylsulfonylamino)-2,2-dimethyl-chroman-8-yl-oxyessigsäure
4-(Phenylsulfonylamino)-2,2-spirocyclopentyl-chroman-8-yl-oxyessigsäure
4-(4-Fluorphenylsulfonylamino)-2,2-spirocyclopentyl-chroman-8-yl-oxyessigsäure
4-(4-Chlorphenylsulfonylamino)-2,2-spirocyclopentyl-chroman-8-yl-oxyessigsäure
4-(4-Methoxyphenylsulfonylamino)-2,2-spirocyclopentyl-chroman-8-yl-oxyessigsäure
4-(4-Fluorphenylsulfonylamino)-2-phenyl-chroman-8-yl-oxyessigsäure
4-(4-Chlorphenylsulfonylamino)-2-phenyl-chroman-8-yl-oxyessigsäure
4-(Phenylsulfonylamino)-chroman-7-yl-essigsäure
4-(4-Fluorphenylsulfonylamino)-chroman-7-yl-essigsäure
4-(4-Chlorphenylsulfonylamino)-chroman-7-yl-essigsäure
4-(3-Fluorphenylsulfonylamino)-chroman-7-yl-essigsäure
4-(4-Methylphenylsulfonylamino)-chroman-7-yl-essigsäure
4-(4-Cyanophenylsulfonylamino)-chroman-7-yl-essigsäure
4-(Phenylsulfonylamino)-chroman-8-yl-essigsäure
4-(4-Fluorphenylsulfonylamino)-chroman-8-yl-essigsäure
4-(4-Chlorphenylsulfonylamino)-chroman-8-yl-essigsäure
4-(4-Methylphenylsulfonylamino)-chroman-8-yl-essigsäure
4-(4-Methoxyphenylsulfonylamino)-chroman-8-yl-essigsäure
4-(4-Trifluormethylphenylsulfonylamino)-chroman-8-yl-essigsäure
4-(3-Fluorphenylsulfonylamino)-chroman-8-yl-essigsäure
4-(4-Fluorphenylsulfonylaminomethyl)-chroman-8-yl-thioessigsäure
4-(4-Fluorphenylsulfonylamino)-2,2-dimethyl-chroman-7-yl-thioessigsäure
4-(4-Chlorphenylsulfonylaminomethyl)-chroman-7-yl-thioessigsäure
4-(4-Fluorphenylsulfonylamino)-chroman-8-yl-thioessigsäure.

Es wurde ferner gefunden, daß man die neuen Chromanderivate der allgemeinen Formel (I) erhält, wenn man die Amine der allgemeinen Formel (II)

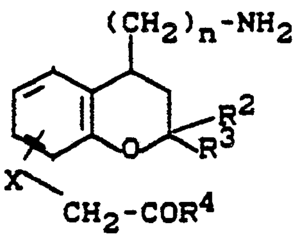

(II)

in denen
$R^2$, $R^3$, $R^4$, X und n die oben angegebene Bedeutung haben,
in an sich bekannter Art und Weise mit Sulfonsäuren der allgemeinen Formel (III)

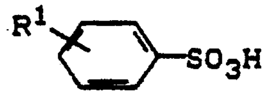

(III)

wobei
$R^1$ die oben angegebene Bedeutung besitzt,
oder deren aktivierte Derivate wie Säurechloride oder aktivierte Ester umsetzt.

Im Falle von $R^4 \neq OH$ schließt sich eine Verseifung zu den freien Carbonsäuren an und dann im Fall der Herstellung der Salze eine Umsetzung mit den entsprechenden Basen.

Die Amine der allgemeinen Formel (II), in denen $R^2$, $R^3$, $R^4$ und X die oben angegebenen Bedeutungen haben und n = 1 ist, sind neu. Man erhält sie aus den entsprechenden $\alpha,\beta$-ungesättigten Nitrilen der Formel (IV)

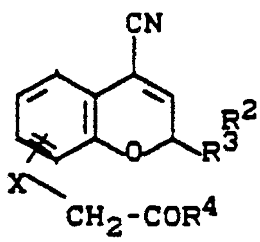

(IV)

in denen
$R^2$, $R^3$, $R^4$ und X die oben genannten Bedeutungen besitzen,
durch katalytische Hydrierung in Gegenwart von $NH_3$. Die Hydrierung kann vorzugsweise nach literaturbekannten Verfahren durchgeführt werden (siehe beispielsweise Rylander, "Catalytic Hydrogenation", Academic Press, Inc., New York, 1967).

Die neuen $\alpha,\beta$-ungesättigten Nitrile (IV) erhält man aus den Chromanonen der allgemeinen Formel (V)

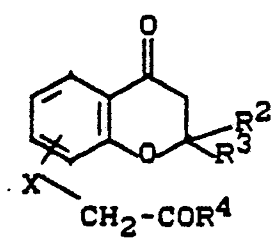

(V)

wobei
$R^2$, $R^3$, $R^4$ und X die oben angegebenen Bedeutungen besitzen,
durch Umsetzung mit Trimethylsilylcyanid, gegebenenfalls in Gegenwart eines Katalysators, wobei die silylierten Cyanhydrine entstehen, die direkt mit Säuren zu den $\alpha,\beta$-ungesättigten Nitrilen der Formel (IV) umgesetzt werden (vgl. Lit.: D.A. Evans, G.L. Carroll, L.H. Truesdale, J. Org. Chem. 39, 914 (1974)).

Die Amine der allgemeinen Formel (II), in denen $R^2$, $R^3$, $R^4$ und X die oben angegebenen Bedeutungen besitzen und n = 0 ist, sind neu. Man erhält sie ebenfalls aus den Chromanonen der allgemeinen Formel (V) durch katalytische reduktive Aminierung analog literaturbekannter Verfahren (z.B. J. Am. Chem. Soc. 93, 2897 (1971); Rylander, "Catalytic Hydrogenation", S. 291-303, Academic Press, Inc. New York, 1967; Org. Reactions 4, 174-255 (1948)) oder durch reduktive Aminierung mit komplexen Metallhydriden (vgl. Harada, in Patai, "The Chemistry of the Carbon-Nitrogen Double Bond", Interscience Publishers, New York, 1970, S. 276-293; J. Am. Chem. Soc. 93, 2897 (1971)).

Die Chromanone der allgemeinen Formel (V), in denen $R^2$, $R^3$ und $R^4$ die oben angegebenen Bedeutungen besitzen und X für Sauerstoff steht, können hergestellt werden aus den Hydroxychromanonen der allgemeinen Formel (VI),

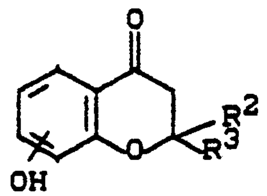

(VI)

in denen
$R^2$ und $R^3$ die oben angegebenen Bedeutungen besitzen,
durch Alkylierung mit Essigsäurederivaten der allgemeinen Formel (VII)

$A-CH_2-COR^4$ (VII)

in welcher
A eine Abgangsgruppe wie z.B. Cl, Br, I, $SO_2CH_3$,

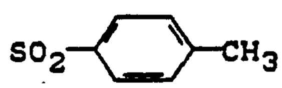

bedeutet und

$R^4$ die oben angegebene Bedeutung besitzt,
nach literaturbekannten Verfahren (z.B. Patai "The Chemistry of the Hydroxyl Group", pt. 1., S. 454-466, Interscience Publishers, New York, 1971; Tetrahedron 30, 1379 (1974)).

Die Hydroxy-4-chromanone der allgemeinen Formel (VI), in denen $R^2$ und $R^3$ die oben angegebenen Bedeutungen besitzen, sind teilweise bekannt oder können durch Etherspaltung analog literaturbekannter Verfahren aus den bekannten entsprechenden Methoxy-4-chromanonen hergestellt werden (z.B. J. M. Lockhart, in Ellis, "Chromenes, Chromanones and Chromones", Interscience Publication, New York, 1977, S. 207-428; J. Org. Chem. 14, S. 366 (1949); H.J. Kabbe, Synthesis 1978, 886).

Die Chromanone der allgemeinen Formel (V), in denen $R^2$, $R^3$ und $R^4$ die oben angegebenen Bedeutungen besitzen und X für eine Bindung steht, sind teilweise bekannt. Man erhält sie aus den entsprechenden Hydroxyphenylessigsäurederivaten der allgemeinen Formel (VIII)

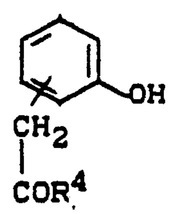

(VIII)

in denen
$R^4$ die oben angegebene Bedeutung besitzt,
nach literaturbekannten Verfahren. Beispielhaft seien genannt:
- die Addition von Acrylnitril an die phenolische Hydroxygruppe und anschließende Cyclisierung - evtl. nach vorhergehender Verseifung zur Carbonsäure und Überführung in das Säurechlorid - unter Friedel-Crafts-Bedingungen. In diesem Fall sind in der allgemeinen Formel (V) $R^2$ und $R^3$ gleich Wasserstoff (z.B. J.M. Lockhart, in Ellis, "Chromenes, Chromanones and Chromones", Interscience Publication, New York, 1977, S. 236-37)

- die Friedel-Crafts Acylierung der Hydroxyphenylessigsäurederivate (VIII) mit Acrylsäurechloriden der allgemeinen Formel (IX)

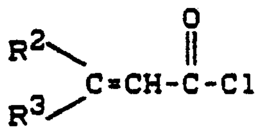

(IX)

in denen

$R^2$ und $R^3$ die oben angegebenen Bedeutungen besitzen

(z.B. J.M. Lockhart in Ellis, "Chromenes, Chromanones and Chromones", Interscience Publication, New York, 1977, S. 237-256)

- die Umsetzung der entsprechend substituierten 2-Hydroxy-acetophenone (IX)

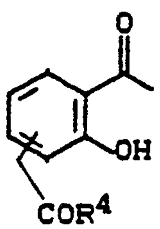

(X)

in denen

$R^4$ die oben angegebene Bedeutung besitzt,

mit Carbonylverbindungen der allgemeinen Formel (XI)

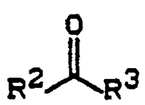

(XI)

in denen

$R^2$ und $R^3$ die oben angegebenen Bedeutungen besitzen,

in Gegenwart von Pyrrolidin (H.J. Kabbe, Synthesis 1978, 886).

Bei der Durchführung des erfindungsgemäßen Verfahrens entstehen im allgemeinen Zwischenprodukte, die isoliert werden können. So ist es möglich, das erfindungsgemäße Verfahren in mehreren Verfahrensstufen auszuführen. Es kann aber auch möglich sein, verschiedene Verfahrensschritte zu kombinieren.

Die Endprodukte I erhält man, indem man die Amine der allgemeinen Formel II mit den entsprechenden Sulfonsäuren der allgemeinen Formel (III), in denen $R^1$ die oben angegebene Bedeutung besitzt, oder deren aktivierte Derivate wie z.B. Sulfonsäurechloride, Sulfonsäureanhydride oder Sulfonsäureester in Gegenwart eines säurebindenden Mittels wie z.B. Alkali- oder Erdalkalihydroxiden oder Alkali- bzw. Erdalkalicarbonaten oder organischen Basen wie z.B. Triethylamin, Pyridin oder N-Ethylmorpholin umsetzt.

Als Lösungsmittel sind in Abhängigkeit von der Art des eingesetzten Sulfonsäurederivats inerte organische Lösungsmittel wie z.B. Methylenchlorid, Chloroform, Essigester, Tetrahydrofuran, Ether, Dimethylformamid und/oder protische Lösungsmittel wie z.B. Wasser, Methanol, Ethanol geeignet.

Die Amine der allgemeinen Formel II, in denen $R^2$, $R^3$, $R^4$ und X die oben angegebene Bedeutung besitzen und n = 1 ist, erhält man durch katalytische Hydrierung der $\alpha,\beta$-ungesättigten Nitrile IV. Als Katalysatoren kommen Metalle wie z.B. Raney-Nickel, Palladium oder Platin in der dem Fachmann bekannten Anwendungsform in Frage. Als Lösemittel können protische Lösungsmittel wie z.B. Methanol, Ethanol und/oder aprotische Lösungsmittel wie z.B. Tetrahydrofuran, Toluol, Dimethylformamid, Methylenchlorid oder Dioxan dienen, denen zweckmäßigerweise flüssiger Ammoniak zugesetzt wird. Die Hydrierung wird bei Drücken zwischen 5 und 300 atm, vorteilhaft zwischen 50 und 150 atm durchgeführt. Die Reaktionstemperatur liegt zwischen 20 und 150 °C, vorteilhaft zwischen 30 und 100 °C, die Reaktionszeit

beträgt 15 Minuten bis 6 Stunden.

Die α,β-ungesättigten Nitrile der allgemeinen Formel IV erhält man aus den Chromanonen V durch Umsetzung mit Trimethylsilylcyanid. Als Katalysatoren dieser Reaktion sind Lewis-Säuren wie z.B. Bortrifluorid-Etherat, Zinkiodid, Zinntetrachlorid, Aluminiumtrichlorid oder organische Basen wie Triethylamin, Pyridin oder auch der Komplex aus 18-Krone-6 und Kaliumcyanid geeignet; die Reaktion kann auch ohne Katalysator durchgeführt werden.

Der Reaktion kann in inerten organischen Lösungsmitteln wie Diethylether, Tetrahydrofuran, Methylenchlorid, Toluol oder auch ohne Lösungsmittel durchgeführt werden.

Die Reaktionstemperatur liegt zwischen 0 und 100° C, bevorzugt zwischen 20 und 60° C.

Die rohen silylierten Cyanhydrine werden durch Behandeln mit Säuren in die α,β-ungesättigten Nitrile IV überführt. Als Säuren kommen in Frage organische Säuren wie z.B. Trifluoressigsäure, p-Toluolsulfonsäure, Ameisensäure, Essigsäure oder Mineralsäuren wie Halogenwasserstoffsäuren, Schwefelsäure oder Salpetersäure. Als Lösungsmittel sind geeignet Alkohole wie z.B. Methanol, Ethanol, Propanol oder nicht protische Lösungsmittel wie Benzol, Toluol, chlorierte Kohlenwasserstoffe, Tetrahydrofuran, Dimethylformamid oder man arbeitet ohne Lösungsmittel.

Die Reaktionstemperatur liegt zwischen 0 und 120° C, bevorzugt zwischen 25 und 100° C, die Reaktionszeit liegt zwischen 10 Minuten und 6 Stunden.

Die Amine der allgemeinen Formel II, in denen $R^2$, $R^3$, $R^4$ und X die oben angegebenen Bedeutungen besitzen und n = 0 ist, erhält man aus den Chromanonen der allgemeinen Formel V durch reduktive Aminierung nach üblichen Methoden.

Als Lösemittel eignen sich bei der Aminierung die üblichen organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Tetrahydrofuran oder Dioxan, oder Chlorkohlenwasserstoffe wie Methylenchlorid oder Chloroform, oder Acetonitril, Dimethylformamid, Dimethylsulfoxid, Eisessig oder Gemische der genannten Lösemittel.

Als Reduktionsmittel eignen sich die üblichen komplexen Hydride wie beispielsweise Natriumborhydrid, Natriumcyanoborhydrid, oder Aminoborankomplexe, oder auch Wasserstoff, gegebenenfalls in Anwesenheit eines Metallkatalysators wie Raney-Nickel oder Palladium.

Als ammoniakhaltige Komponente werden wäßrige Ammoniaklösung, gasförmiger Ammoniak, oder auch Ammoniumsalze wie Ammoniumchlorid, Ammoniumsulfat, Ammoniumacetat oder Ammoniumformiat eingesetzt.

Die Umsetzung kann bei normalem, erhöhtem oder bei erniedrigtem Druck durchgeführt werden (0,5 bis 5 bar). Im allgemeinen arbeitet man bei der Verwendung von komplexen Metallhydriden bei normalem Druck, bei der Verwendung von Wasserstoff mit Überdruck.

Die reduktive Aminierung wird im allgemeinen in einem Temperaturbereich von 0° C bis +150° C, bevorzugt von +20° C bis +100° C durchgeführt.

Ausgehend von den Chromanen V kann die Synthese der Endprodukte I in folgenden Reaktionsschemata zusammengefaßt werden:

a) für n = 1 in der allgemeinen Formel I

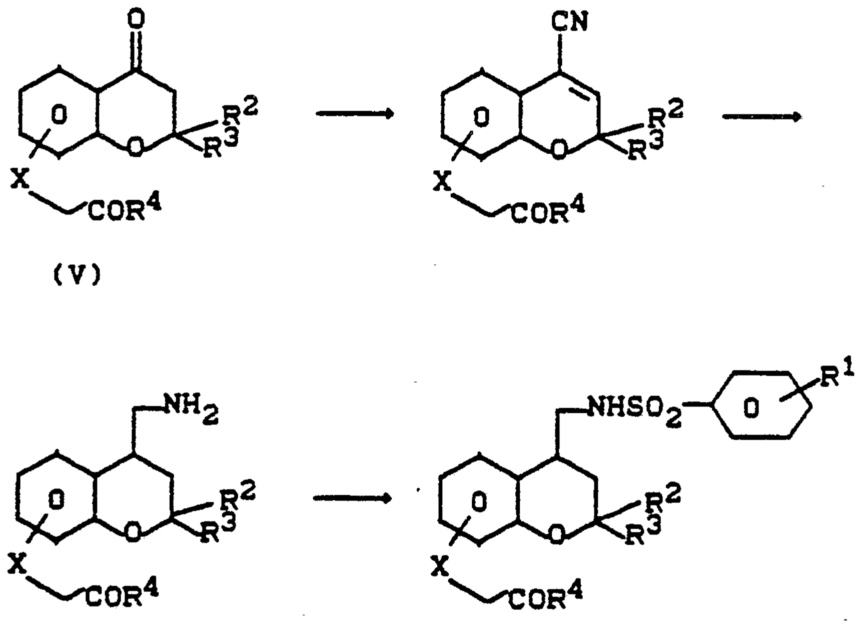

(V)

b) für n = 0 in der allgemeinen Formel I

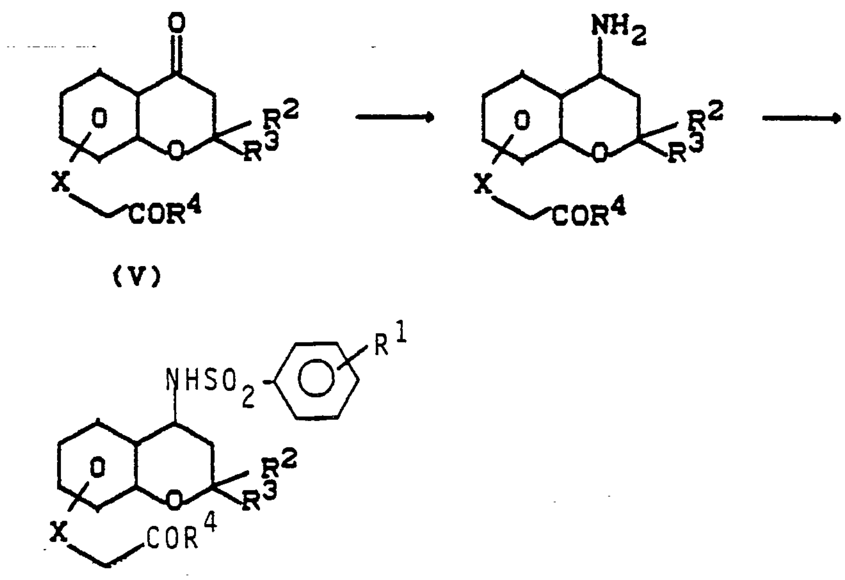

(V)

Als Ausgangsaminochromane der allgemeinen Formel II seien beispielhaft genannt:

4-Aminomethyl-7-chromanyl-essigsäure
2,2-Dimethyl-4-aminomethyl-7-chromanyl-essigsäure
2,2-Spirocyclopentyl-4-aminomethyl-7-chromanyl-essigsäure
4-Aminomethyl-8-chromanyl-aessigsäure
2,2-Dimethyl-4-aminomethyl-8-chromanyl-essigsäure
2,2-Spirocyclopentyl-4-aminomethyl-8-chromanyl-essigsäure
2,2-Spirocyclohexyl-4-aminomethyl-8-chromanyl-essigsäure
2-Phenyl-4-aminomethyl-8-chromanyl-essigsäure
4-Aminomethyl-8-chromanyl-oxyessigsäure
2,2-Dimethyl-4-aminomethyl-8-chromanyl-oxyessigsäure
2,2-Diethyl-4-aminomethyl-8-chromanyl-oxyessigsäure
2,2-Spirocyclohexyl-4-aminomethyl-8-chromanyl-oxyessigsäure
2-Ethyl-4-aminomethyl-8-chromanyl-oxyessigsäure

4-Amino-7-chromanyl-oxyessigsäure
2,2-Dimethyl-4-amino-7-chromanyl-oxyessigsäure
2,2-Spirocyclopentyl-4-amino-7-chromanyl-oxyessigsäure
4-Aminomethyl-7-chromanyl-oxyessigsäure
2,2-Dimethyl-4-aminomethyl-7-chromanyl-oxyessigsäure
2,2-Diethyl-4-aminomethyl-7-chromanyl-oxyessigsäure
2,2-Spirocyclopentyl-4-aminomethyl-7-chromanyl-oxyessigsäure
2,2-Spirocyclohexyl-4-aminomethyl-7-chromanyl-oxyessigsäure
2-Phenyl-4-aminomethyl-7-chromanyl-oxyessigsäure
4-Amino-8-chromanyl-oxyessigsäure
2,2-Dimethyl-4-amino-8-chromanyl-oxyessigsäure
2,2-Spirocyclopentyl-4-amino-8-chromanyl-oxyessigsäure
2-Phenyl-4-amino-8-chromanyl-oxyessigsäure
4-Amino-7-chromanyl-essigsäure
2,2-Dimethyl-4-amino-7-chromanyl-essigsäure
2,2-Spirocyclopentyl-4-amino-7-chromanyl-essigsäure
4-Amino-8-chromanyl-essigsäure
2,2-Dimethyl-4-amino-8-chromanyl-essigsäure
2,2-Spirocyclopentyl-4-amino-8-chromanyl-essigsäure

Die Ausgangsaminochromane der allgemeinen Formel II sind neu.

Die neuen substituierten Chromanylderivate bzw. deren Salze können als Wirkstoffe in Arzneimitteln eingesetzt werden. Die Wirkstoffe weisen eine thrombozytenaggregationshemmende sowie eine vasodilatorische und eine bronchodilatorische Wirkung auf. Sie können bevorzugt zur Behandlung von Thrombosen, Thromboembolien, Ischämien, als Antiasthmatika und als Antiallergika eingesetzt werden. Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B.: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in

mehreren Einzelgaben über den Tag zu verteilen.

Methodik

Thrombozytenaggregationshemmung in vitro

Für die in vitro-Bestimmung der thrombozytenaggregationshemmenden Wirkung wird Blut von gesunden Spendern, die mindestens 14 Tage lang kein Medikament eingenommen hatten, verwendet. Das Blut wird in 3,8 %iger Natriumcitratlösung aufgenommen. Plättchenreiches Plasma (PRP) wird durch 20 Min. lange Zentrifugation bei 150 g bei Raumtemperatur gewonnen (Jürgens/Beller: Klinische Methoden der Blutgerinnungsanalyse; Thieme Verlag, Stuttgart 1959). Die Plättchenaggregation wird nach der turbidometrischen Methode (Born, G.V.R.: J. Physiol. 162, 67, 1962) im Aggregometer bei 37 °C bestimmt. Hierzu wird PRP mit der Prüfsubstanz bei 37 °C inkubiert und anschließend die Aggregation durch Zugabe eine Kollagensuspension ausgelöst. Für die in-vitro-Versuche wird die minimal effektive Wirkstoffkonzentration (MEK) angegeben, die in den entsprechenden PRP-proben die Thrombozytenaggregation hemmt.

Tabelle

| Beispiel Nr. | Thrombozytenaggregationshemmung (in vitro)/Minimal effektive Wirkstoffkonzentration (MEK) |
|---|---|
| 28 | < 0,03 |
| 29 | < 0,03 |
| 30 | 0,01 - 0,03 |
| 31 | 0,3 |
| 32 | 3 - 10 |
| 33 | 10 - 30 |
| 34 | 0,01 - 0,1 |
| 35 | 0,03 - 0,01 |
| 36 | 0,03 - 0,1 |
| 37 | 0,1 - 0,3 |
| 38 | 0,03 - 0,1 |
| 39 | 1 - 10 |

Thrombozytenaggregationshemmung ex vivo

Für die ex vivo-Untersuchungen wird den Tieren die Wirksubstanz in einer Tylosesuspension oral verabreicht. Nach 90 Minuten werden die Tiere entblutet und das PRP mittels Zentrifugation gewonnen. Die Messung der Aggregationshemmung erfolgt analog dem Verfahren, welches für die in vitro-Versuche beschrieben ist; jedoch ohen Vorinkubation der Proben.

Herstellungsbeispiele

Beispiel 1

4-Cyano-chrom-3-en-7-yl-essigsäuremethylester

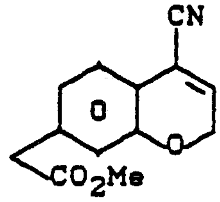

5,85 g (25 mmol) Chroman-4-on-7-yl-essigsäuremethylester und 7,43 g (75 mmol) Trimethylsilylcyanid werden mit einigen Tropfen $BF_3$-Etherat versetzt und bei Raumtemperatur gerührt bis im IR die Ketonbande bei 1690 $cm^{-1}$ verschwunden ist (ca. 45 Min). Es wird 25 ml Trifluoressigsäure zugesetzt und 2 h am Rückfluß gekocht. Die Trifluoressigsäure wird im Wasserstrahlvakuum abgezogen, der Rückstand in 50 ml Methylenchlorid aufgenommen und mit gesättigter Natriumhydrogencarbonatlösung neutral gewaschen. Danach wird einmal mit gesättigter Natriumchloridlösung gewaschen, über Natriumsulfat getrocknet, das Trockenmittel abfiltriert und eingedampft. Der Rückstand wird aus Ligroin umkristallisiert. Fp.: 65 °C; Ausbeute: 3,55 g (41,5 % der Theorie)

Beispiel 2

2,2-Dimethyl-4-cyano-chrom-3-en-7-yl-essigsäuremethylester

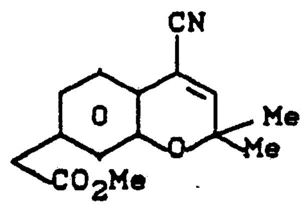

Darstellung aus 2,2-Dimethyl-chroman-4-on-7-yl-essigsäuremethylester analog Beispiel 1.
Fp.: 61 °C, Ausbeute: 53,3 % der Theorie.

Beispiel 3

4-Cyano-chrom-3-en-8-yl-oxyessigsäuremethylester

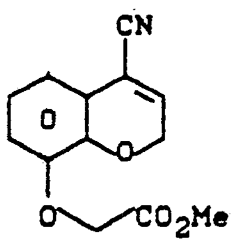

Darstellung aus Chroman-4-on-8-yl-oxyessigsäuremethylester analog Beispiel 1.
Fp.: 94-96 °C, Ausbeute: 57,5 % der Theorie.

Beispiel 4

4-Cyano-chrom-3-en-7-yl-oxyessigsäuremethylester

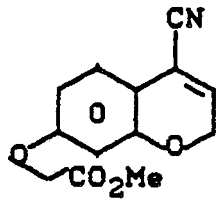

Darstellung aus Chroman-4-on-7-yl-oxyessigsäuremethylester analog Beispiel 1.
Fp.: 104 ° C, Ausbeute: 67%.

Beispiel 5

2,2-Dimethyl-4-cyano-chrom-3-en-7-yl-oxyessigsäuremethylester

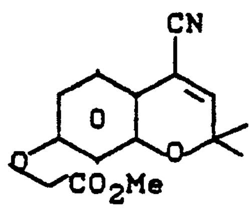

Darstellung aus 2,2-Dimethyl-chroman-4-on-7-yl-oxyessigsäuremethylester analog Beispiel 1.
Fp.: 78-79 ° C, Ausbeute: 71,6 % der Theorie.

Beispiel 6

2,2-Spirocyclopentyl-4-cyano-chrom-3-en-7-yl-oxyessigsäuremethylester

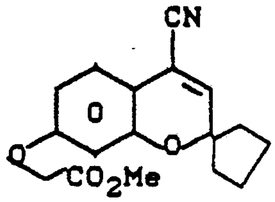

Darstellung aus 2,2-Spirocyclopentyl-chroman-4-on-7-yl-oxyessigsäuremethylester analog Beispiel 1.
Fp.: 81 ° C, Ausbeute: 68,7 % der Theorie.

Beispiel 7

2,2-Spirocyclohexyl-4-cyano-chrom-3-en-7-yl-oxyessigsäuremethylester

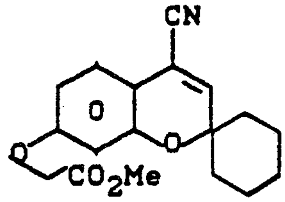

Darstellung aus 2,2-Spirocyclohexyl-chroman-4-on-7-yl-oxyessigsäuremethylester analog Beispiel 1. Fp.: 72-75° C, Ausbeute: 59,8 % der Theorie.

Beispiel 8

4-Aminomethyl-chroman-7-yl-essigsäureamid

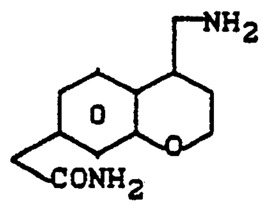

9,93 g (43,4 mmol) Nitril aus Beispiel 1 werden in 100 ml Methanol und 80 ml flüssigem Ammoniak in Gegenwart von 5 g Raney-Nickel 4,5 h bei 70° C und 100 atm. mit Wasserstoff hydriert. Der Katalysator wird abfiltriert, einmal mit 100 ml Methanol ausgekocht und die vereinigten Methanollösungen eingedampft. Der Rückstand wird an Kieselgel mit einem Gemisch von Methylenchlorid und Methanol im Verhältnis von 10:1 als Eluens bei 2 bar chromatographiert. Nach Eindampfen des Laufmittels bleibt das Produkt als Schaum zurück.
IR ($CHCl_3$): 1655 $cm^{-1}$ (Amid); $^1$H-NMR ($CDCl_3$): $\delta$ = 3,4 (s, 2H) ($CH_2$ Gruppe neben Amid).
Ausbeute: 4,37 g (48,6 %)

Beispiel 9

2,2-Dimethyl-4-aminomethyl-chroman-7-yl-essigsäureamid

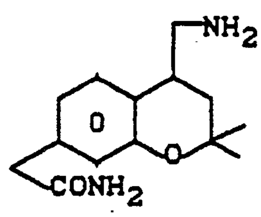

Darstellung analog Beispiel 9.
Der nach der Chromatographie erhaltene, nicht umkristallisierte Schaum schmilzt bei 161° C. IR ($CHCl_3$):1655 $cm^{-1}$; $^1$H-NMR ($CDCl_3$): $\delta$ = 3,4 (s,2H).
($CH_2$-Gruppe neben Amid).
Ausbeute: 40,7 % der Theorie.

Beispiel 10

4-Amino-chroman-8-yl-oxyessigsäureamid

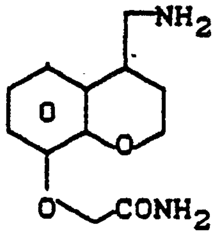

Darstellung analog Beispiel 8.
Chromatographiert mit Aceton/Methanol im Verhältnis 5:1.
IR ($CHCl_3$): 1670 $cm^{-1}$ (Amid); $^1$H-NMR ($CDCl_3$): $\delta$ = 4,45 (s,2H) ($CH_2$-Gruppe neben Amid).
Ausbeute: 53,1 % der Theorie.

Beispiel 11

4-Aminomethyl-chroman-7-yl-oxyessigsäureamid

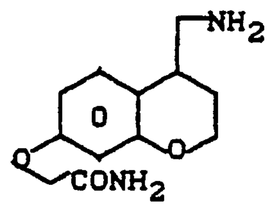

Darstellung analog Beispiel 8.
IR ($CHCl_3$): 1675 $cm^{-1}$ (Amid); $^1$H-NMR ($CDCl_3$) $\delta$ = 4,45 (s,2H) ($CH_2$-Gruppe neben Amid).
Ausbeute: 58,2 % der Theorie.

Beispiel 12

2,2-Dimethyl-4-aminomethyl-chroman-7-yl-oxyessigsäureamid

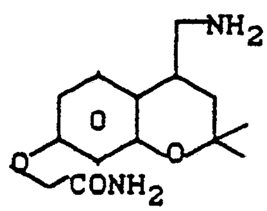

Darstellung analog Beispiel 8.
IR ($CHCl_3$): 1675 $cm^{-1}$; (Amid); $^1$H-NMR ($CDCl_3$) $\delta$ = 4,45 (s.2H) ($CH_2$-Gruppe neben Amid).
Ausbeute: 75,8 % der Theorie.

Beispiel 13

2,2-Spirocyclopentyl-4-aminomethyl-chroman-7-yl-oxyessigsäureamid

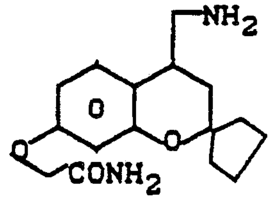

Darstellung analog Beispiel 8.
IR ($CHCl_3$) :1675 $cm^{-1}$ (Amid); $^1$H-NMR ($CDCl_3$) $\delta$ = 4,47 (s,2H) ($CH_2$-Gruppe neben Amid); $\delta$ = 1,4-2,1 (m, 12H)
Ausbeute: 71,5 % der Theorie.

Beispiel 14

2,2-Spirocyclohexyl-4-aminomethyl-chroman-7-yl-oxyessigsäureamid

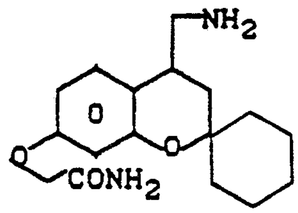

Darstellung analog Beispiel 8.
IR ($CHCl_3$): 1675 $cm^{-1}$ (Amid); $^1$H-NMR ($CDCl_3$): $\delta$ = 4,4 (s,2H ($CH_2$-Gruppe neben Amid), $\delta$ = 1,35-2,1 (m, 14H).

Beispiel 15

2,2-Dimethyl-4-amino-chroman-7-yl-oxyessigsäureamid

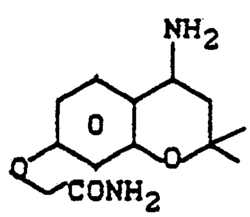

50 g (0,2 Mol) 2,2-Dimethyl-chroman-4-on-7-yl-oxyessigsäuremethylester werden in 200 ml Methanol und 250 ml flüssigem Ammoniak in Gegenwart von 15 g Raney-Nickel und 1 g Ammoniumacetat 11 h bei 110 °C und 100 bar $H_2$ reduktiv aminiert. Der Katalysator wird abfiltriert und mit Methanol ausgekocht. Die weitere Aufarbeitung erfolgt analog Beispiel 8.
IR ($CHCl_3$): 1675 $cm^{-1}$; (Amid); $^1$H-NMR ($CDCl_3$): $\delta$ = 1,25 (s,3H); 1,4 (s, 3H) (Methylgruppen in 2-Stellung); $\delta$ = 4,45 ($CH_2$-Grupppe neben Amid). Fp.: 125 °C (aus Toluol).
Ausbeute: 23,55 g (47,1 %).

Beispiel 16

4-(4-Chlorphenylsulfonylaminomethyl)-chroman-7-yl-essigsäureamid

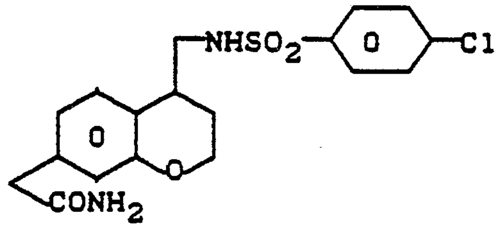

2,48 g (10 mmol) 4-Aminomethyl-chroman-7-yl-essigsäureamid werden zusammen mit 2,53 g (12 mmol) 4-Chlorbenzolsulfonsäurechlorid in 10 ml Pyridin, dem eine Spatelspitze Dimethylaminopyridin zugesetzt ist, 2 bis 3 h bei Raumtemperatur gerührt. Die Lösung wird auf 50 ml eiskalte 10 % HCl gegeben und dreimal mit Essigester ausgeschüttelt. Die vereinigten Essigesterphasen werden einmal mit 10 % HCl, 1 mal mit gesättigter $NaHCO_3$ Lösung und 1 mal mit gesättigter NaCl Lösung gewaschen, über Natriumsulfat getrocknet und eingedampft. Der Rückstand wird an Kieselgel mit Methylenchlorid-Methanol im Verhältnis 10:1 chromatographiert. Nach dem Abdampfen des Eluens bleibt das Produkt als Feststoff zurück.
Fp.: 165-70° C (nicht umkristallisert); $^1$H-NMR ($CDCl_3$): $\delta$ = 3,45 (s, 2H), Ausbeute: 2,48 g (63 % der Theorie).

Beispiel 17

4-(4-Fluorphenylsulfonylaminomethyl)-chroman-7-yl-essigsäureamid

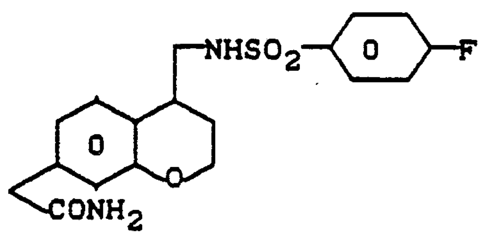

Darstellung aus 4-Aminomethyl-chroman-7-yl-essigsäureamid und 4-Fluorbenzolsulfonsäurechlorid analog Beispiel 16.
$^1$H-NMR ($CDCl_3$): $\delta$ = 3,48 (s, 2H)
Ausbeute: 58,3 % der Theorie.

Beispiel 18

4-(4-Chlorphenylsulfonylaminomethyl)-2,2-dimethyl-chroman-7-yl-essigsäureamid

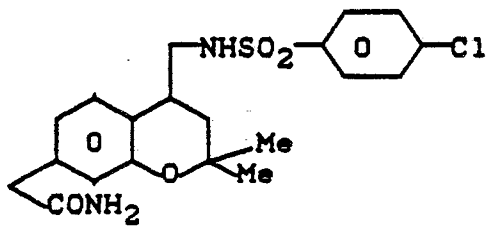

Darstellung aus 2,2-Dimethyl-4-aminomethyl-chroman-7-yl-essigsäureamid und 4-Chlorbenzolsulfonsäurechlorid analog Beispiel 16.
Fp.: 74-78° C (nicht umkristallisiert).
Ausbeute: 67,9 % der Theorie.

Beispiel 19

4-(4-Fluorphenylsulfonylaminomethyl)-chroman-8-yl-oxyessigsäureamid

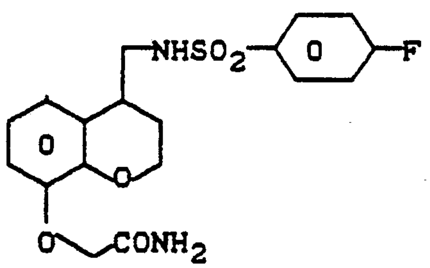

Darstellung aus 4-Aminomethyl-chroman-8-yl-oxyessigsäureamid und 4-Fluorbenzolsulfonsäurechlorid analog Beispiel 16.
Fp.: 137° C (nicht umkristallisiert).
Ausbeute: 41,9 % der Theorie.

Beispiel 20

4-(4-Chlorphenylsulfonylamino)-2,2-dimethyl-chroman-7-yl-oxyessigsäureamid

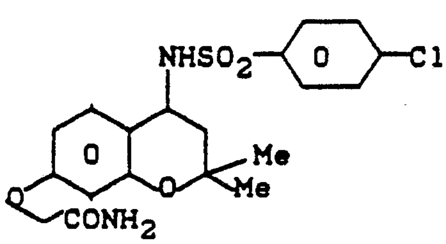

Darstellung aus 4-Amino-2,2-dimethyl-chroman-7-yl-oxyessigsäureamid und 4-Chlorbenzolsulfonsäurechlorid analog Beispiel 16. Fp.: 177-180° C (nach Chromatographie aus Toluol/Petrolether umkristallisiert).
Ausbeute: 48.6 % der Theorie.

Beispiel 21

4-(4-Fluorphenylsulfonylamino)-2,2-dimethyl-chroman-7-yl-oxyessigsäureamid

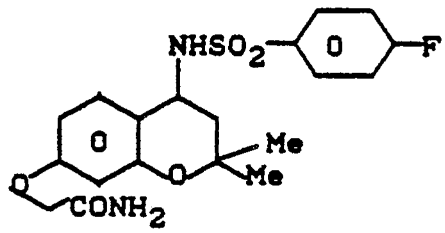

Darstellung aus 4-Amino-2,2-dimethyl-chroman-7-yl-oxyessigsäureamid und 4-Fluorbenzolsulfonsäurechlorid analog Beispiel 16.
Fp.: 191 °C (nach Chromatographie aus Toluol/Petrolether umkristallisiert).
Ausbeute: 33,8 % der Theorie.

Beispiel 22

4-(4-Chlorphenylsulfonylaminomethyl)-chroman-7-yl-oxyessigsäureamid

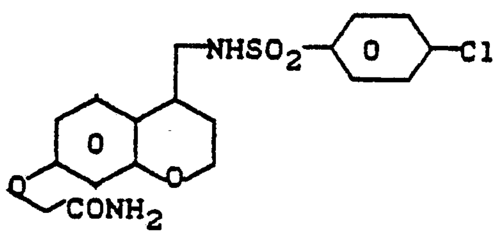

Darstellung aus 4-Aminomethyl-chroman-7-yl-oxyessigsäureamid und 4-Chlorbenzolsulfonsäurechlorid analog Beispiel 16.
Fp.: 80-85 °C (nicht umkristallisiert).
Ausbeute: 58,2 % der Theorie.

Beispiel 23

4-(4-Chlorphenylsulfonylaminomethyl)-2,2-dimethyl-chroman-7-yl-oxyessigsäureamid

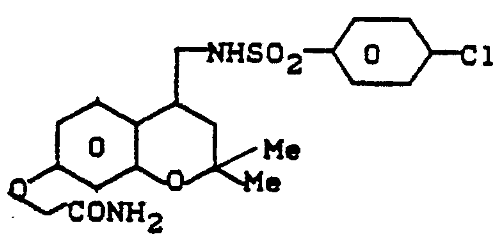

Darstellung aus 4-Aminomethyl-2,2-dimethyl-chroman-7-yl-oxyessigsäureamid und 4-Chlorbenzolsulfonsäurechlorid analog Beispiel 16.
Fp.: 178° C (nach Chromatographie aus Toluol ausgekocht, Produkt bleibt sauber ungelöst zurück).
Ausbeute: 62,9 % der Theorie.

Beispiel 24

4-(4-Fluorphenylsulfonylaminomethyl)-2,2-dimethyl-chroman-7-yl-oxyessigsäureamid

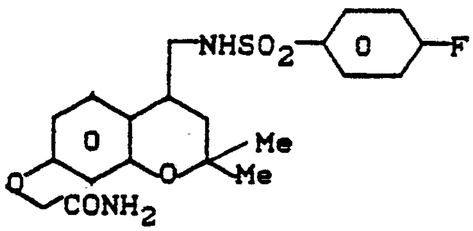

Darstellung aus 4-Aminomethyl-2,2-dimethyl-chroman-7-yl-oxyessigsäureamid und 4-Fluorbenzolsulfonsäurechlorid analog Beispiel 16.
IR (KBr): 1680 $cm^{-1}$ (CON); 1330 $cm^{-1}$ ($SO_2N$); 1170 $cm^{-1}$ ($SO_2N$).
Ausbeute: 71,8 % der Theorie.

Beispiel 25

4-(4-Methylphenylsulfonylaminomethyl)-2,2-dimethyl-chroman-7-yl-oxyessigsäureamid

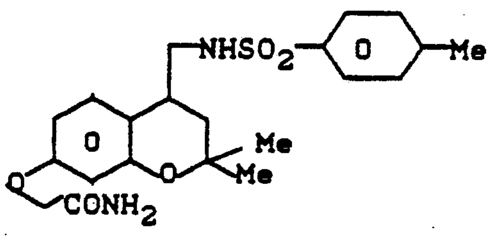

Darstellung aus 4-Aminomethyl-2,2-dimethyl-chroman-7-yl-oxyessigsäureamid und 4-Methylbenzolsulfonsäurechlorid analog Beispiel 16.
IR (KBr): 1660 $cm^{-1}$ (CON); 1320 $cm^{-1}$ ($SO_2N$); 1170 $cm^{-1}$ ($SO_2N$).
Ausbeute: 60,2 % der Theorie.

Beispiel 26

4-(4-Chlorphenylsulfonylaminomethyl)-2,2-spirocyclopentyl-chroman-7-yl-oxyessigsäureamid

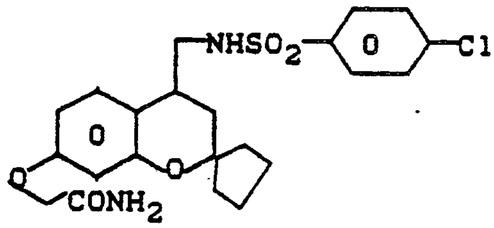

Darstellung aus 4-Aminomethyl-2,2-spirocyclopentyl-chroman-7-yl-oxyessigsäureamid und 4-Chlorbenzolsulfonsäurechlorid analog Beispiel 16.
IR ($CH_2Cl_2$): 1690 $cm^{-1}$ (CON); 1340 $cm^{-1}$ ($SO_2N$); 1160 $cm^{-1}$ ($SO_2N$).
Ausbeute: 61,9 % der Theorie.

Beispiel 27

4-(4-Chlorphenylsulfonylaminomethyl)-2,2-spirocyclohexyl-chroman-7-yl-oxyessigsäureamid

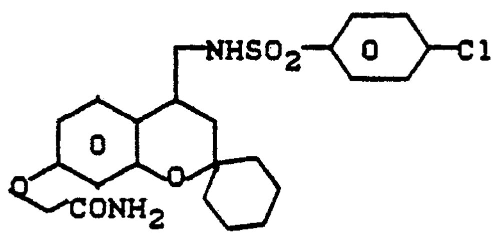

Darstellung aus 4-Aminomethyl-2,2-spirocyclohexyl-chroman-7-yl-oxyessigsäureamid und 4-Chlorbenzolsulfonsäurechlorid analog Beispiel 16.
IR ($CH_2Cl_2$): 1690 $cm^{-1}$ (CON); 1330 $cm^{-1}$ ($SO_2N$); 1160 $cm^{-1}$ ($SO_2N$).
Ausbeute: 70,8 % der Theorie.

Beispiel 28

4-(4-Chlorphenylsulfonylaminomethyl)-chroman-7-yl-essigsäure

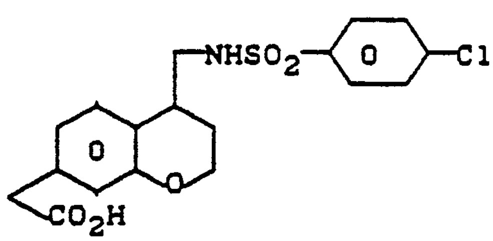

3,945 g (10 mmol) 4-(4-Chlorphenylsulfonylaminomethyl)-chroman-7-yl-essigsäureamid und 25 ml 1n KOH werden nach Zugabe von 50 ml Methanol 5 Stunden refluxiert. Das Methanol wird im Wasserstrahlvakuum abgedampft, die wäßrige Phase einmal mit Essigester ausgeschüttelt und dann mit konzentrierter Salzsäure auf pH 1-2 gebracht. Der Niederschlag wird abfiltriert, die wäßrige Phase wird zweimal mit Essigester ausgeschüttelt, die vereinigten Essigesterphasen werden über Natriumsulfat getrocknet und dann eingedampft. Der Rückstand wird mit dem abfiltrierten Niederschlag vereinigt und am Hochvakuum

getrocknet. Die Säuren sind ohne Umkristallisation oder Chromatographie sauber.
IR (Film): 1710 $cm^{-1}$ (COOH); $^1$H-NMR ($CD_3OD$): δ = 3,4 (s, 2H) ($CH_2$-Gruppe neben COOH).
Ausbeute: 3,28 g (83 % der Theorie).

Beispiel 29

4-(4-Fluorphenylsulfonylaminomethyl)-chroman-7-yl-essigsäure

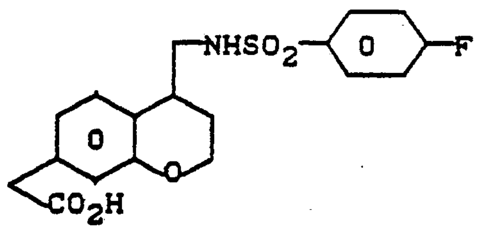

Darstellung durch Verseifung aus dem entsprechenden Amid analog Beispiel 28.
Fp.: 57-60° C (nicht umkristallisiert); $^1$H-NMR ($CD_3OD$): δ = 3,4 (s, 2H) ($CH_2$-Gruppe neben COOH).
Ausbeute: 80,7 % der Theorie.

Beispiel 30

4-(4-Chlorphenylsulfonylaminomethyl)-2,2-dimethyl-7-yl-essigsäure

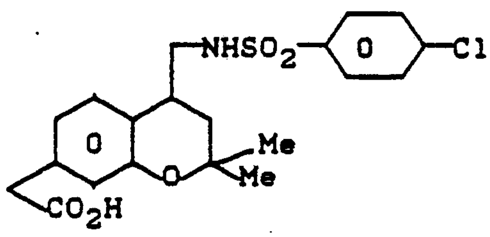

Darstellung durch Verseifung aus dem entsprechenden Amid analog Beispiel 28.
Fp.: 65-70° C (nicht umkristallisiert); $^1$H-NMR ($CD_3OD$): δ = 3,4 (s, 2H) ($CH_2$-Gruppe neben COOH).
Ausbeute: 77,7 % der Theorie.

Beispiel 31

4-(4-Fluorphenylsulfonylaminomethyl)-chroman-8-yl-oxyessigsäure

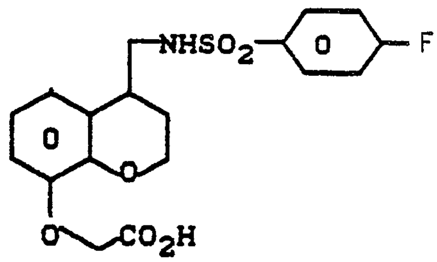

Darstellung durch Verseifung aus dem entsprechenden Amid analog Beispiel 28.
Fp.: 110° C (nicht umkristallisiert); IR (KBr): 1740 $cm^{-1}$ (COOH).
Ausbeute: 76,4 % der Theorie.

Beispiel 32

4-(4-Chlorphenylsulfonylamino)-2,2-dimethyl-chroman-7-yl-oxyessigsäure

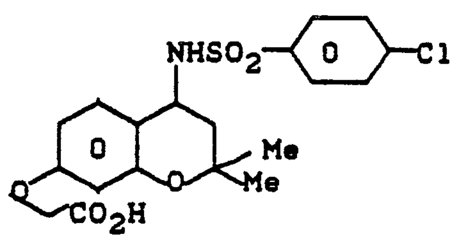

Darstellung durch Verseifung aus dem entsprechenden Amid analog Beispiel 28.
Fp.: 167° C (umkristallisiert aus Toluol/Petrolether) Ausbeute: 58,9 % der Theorie.

Beispiel 33

4-(4-Fluorphenylsulfonylamino)-2,2-dimethyl-chroman-7-yl-oxyessigsäure

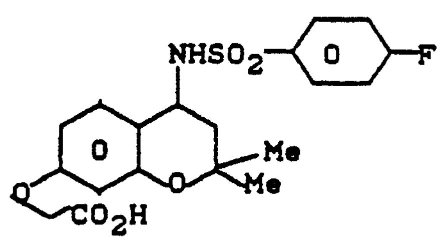

Darstellung durch Verseifung aus dem entsprechenden Amid analog Beispiel 28.
Fp.: 174° C (umkristallisiert aus Toluol/Petrolether) Ausbeute: 94 % der Theorie.

Beispiel 34

4-(4-Chlorphenylsulfonylaminomethyl)-chroman-7-yl-oxyessigsäure

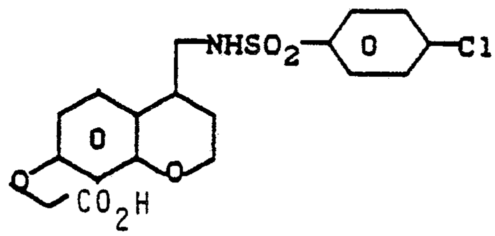

Darstellung durch Verseifung aus dem entsprechenden Amid analog Beispiel 28.
$^1$H-NMR ($CDCl_3$): $\delta$ = 4,6 (s, 2H) ($CH_2$-Gruppe neben COOH);
IR ($CH_2Cl_2$): 1740 $cm^{-1}$ (COOH)
Ausbeute: 95,9 % der Theorie.

Beispiel 35

4-(4-Chlorphenylsulfonylaminomethyl)-2,2-dimethyl-chroman-7-yl-oxyessigsäure

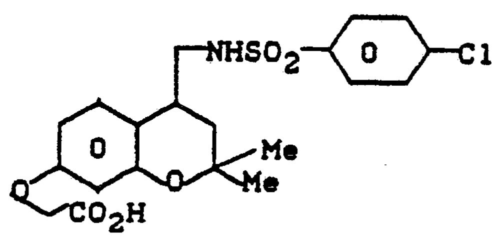

Darstellung durch Verseifung aus dem entsprechenden Amid analog Beispiel 28.
$^1$H-NMR ($CD_3OD$): $\delta$ = 4,5 (s, 2H) ($CH_2$-Gruppe neben COOH);
IR (KBr): 1725 $cm^{-1}$ (COOH)
Ausbeute: 98,9 % der Theorie.

Beispiel 36

4-(4-Fluorphenylsulfonylaminomethyl)-2,2-dimethyl-chroman-7-yl-oxyessigsäure

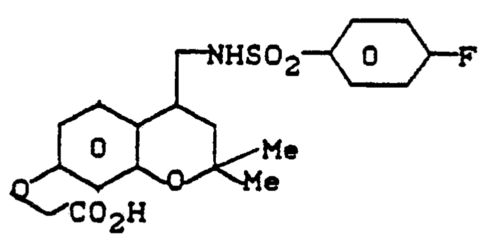

Darstellung durch Verseifung aus dem entsprechenden Amid analog Beispiel 28.
Fp.: 173 °C (umkristallisiert aus Essigester/Petrolether)
Ausbeute: 74,5 % der Theorie.

Beispiel 37

4-(4-Methylphenylsulfonylaminomethyl)-2,2-dimethyl-chroman-7-yl-oxyessigsäure

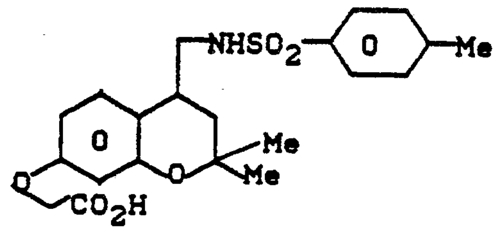

Darstellung durch Verseifung aus dem entsprechenden Amid analog Beispiel 28.
Fp.: 123° C (umkristallisiert aus Essigester/Petrolether)
Ausbeute: 87,1 % der Theorie.

Beispiel 38

4-(4-Chlorphenylsulfonylaminomethyl)-2,2-spirocyclopentyl-chroman-7-yl-oxyessigsäure

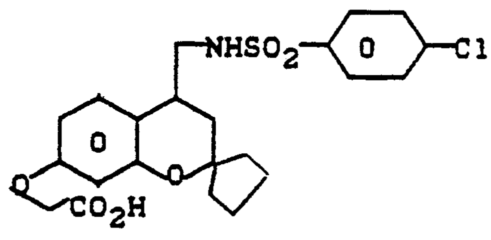

Darstellung durch Verseifung aus dem entsprechenden Amid analog Beispiel 28.
$^1$H-NMR ($CDCl_3$): $\delta$ = 4,6 (s, 2H) ($CH_2$-Gruppe neben COOH);
IR ($CH_2Cl_2$ Film): 1735 $cm^{-1}$ (COOH)
Ausbeute: 80,1 % der Theorie.

Beispiel 39

4-(4-Chlorphenylsulfonylaminomethyl)-2,2-spirocyclohexyl-chroman-7-yl-oxyessigsäure

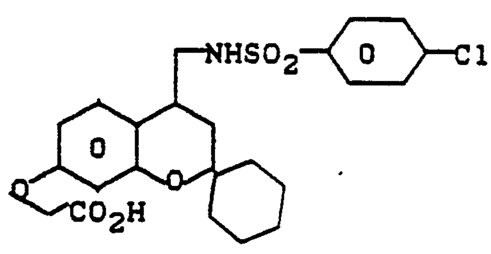

Darstellung durch Verseifung aus dem entsprechenden Amid analog Beispiel 28.
Fp.: 64-68° C (nicht umkristallisiert).
$^1$H-NMR ($CD_3OD$): $\delta$ = 4,6 (s, 2H) ($CH_2$-Gruppe neben COOH);
Ausbeute: 95,1 % der Theorie.

## Ansprüche

1. Chromanderivate der allgemeinen Formel (I)

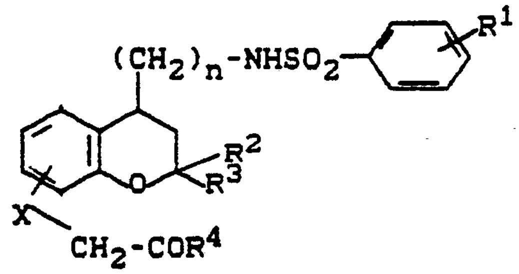

in welcher
$R^1$ für Wasserstoff, Halogen, Alkyl, Hydroxy, Alkoxy, Trifluormethyl oder Cyano steht,
$R^2$ und $R^3$ gleich oder verschieden sind und für Wasserstoff, Alkyl, Cycloalkyl oder Aryl stehen,
$R^4$ für Hydroxy, Alkoxy, Aryloxy, Aralkoxy oder für eine Gruppe der Formel $-NR^5R^6$ steht, wobei $R^5$ und $R^6$ gleich oder verschieden sind und jeweils Wasserstoff, Alkyl, Aryl oder Aralkyl bedeuten,
X eine direkte Bindung oder ein Sauerstoff- oder Schwefelatom, NH oder N-Alkyl bedeutet, und
n 0 oder 1 bedeutet,
sowie deren physiologisch verträglichen Salze.

2. Verbindungen der allgemeinen Formel (I) gemäß Anspruch 1, in welcher
$R^1$ für Wasserstoff, Fluor, Chlor, Brom, Jod, Methyl, Ethyl, Propyl, Isopropyl, Hydroxy, Methoxy, Ethoxy, Propoxy, Isopropoxy, Trifluormethyl oder Cyano steht,
$R^2$ und $R^3$ gleich oder verschieden sind und für Wasserstoff, Methyl, Ethyl, Propyl, Isopropyl, Cyclobutyl, Cyclopentyl, Cyclohexyl, Cycloheptyl oder Phenyl stehen,
$R^4$ für Hydroxy, Methoxy, Ethoxy, Phenoxy, Benzyloxy oder für eine Gruppe der Formel $-NR^5R^6$ steht, wobei $R^5$ und $R^6$ gleich oder verschieden sind und jeweils Wasserstoff, Methyl, Ethyl, Phenyl oder Benzyl bedeuten,
X für eine direkte Bindung oder ein Sauerstoff-oder Schwefelatom steht,
n für 0 oder 1 steht
und deren Salze.

3. Verbindungen der allgemeinen Formel (I) gemäß Anspruch 1 zur Verwendung bei der Bekämpfung von Erkrankungen.

4. Verfahren zur Herstellung von Verbindungen der allgemeinen Formel (I)

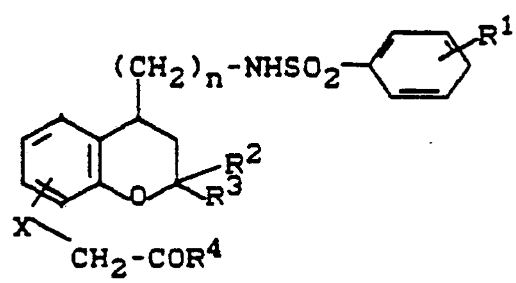

in welcher
$R^1$ für Wasserstoff, Halogen, Alkyl, Hydroxy, Alkoxy, Trifluormethyl oder Cyano steht,
$R^2$ und $R^3$ gleich oder verschieden sind und für Wasserstoff, Alkyl, Cycloalkyl oder Aryl stehen,
$R^4$ für Hydroxy, Alkoxy, Aryloxy, Aralkoxy oder für eine Gruppe der Formel $-NR^5R^6$ steht, wobei $R^5$ und $R^6$ gleich oder verschieden sind und jeweils Wasserstoff, Alkyl, Aryl oder Aralkyl bedeuten,
X eine direkte Bindung oder ein Sauerstoff- oder Schwefelatom, NH oder N-Alkyl bedeutet, und
n 0 oder 1 bedeutet,

sowie deren physiologisch verträglichen Salze, dadurch gekennzeichnet, daß man Amine der allgemeinen Formel (II)

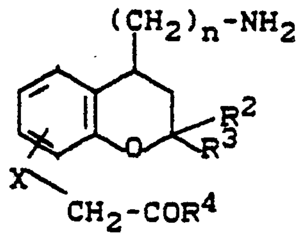

in welcher
$R^2$, $R^3$, $R^4$, X und n die oben angegebene Bedeutung haben, mit Sulfonsäuren der allgemeinen Formel (III)

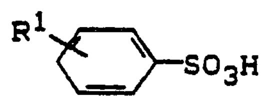

in welcher
R' die oben angegebene Bedeutung hat, oder mit deren aktivierten Derivaten Säurechloriden oder aktivierten Estern umsetzt, und für den Fall, daß $R^4$ nicht Hydroxyl bedeutet, die Säurederivate zu freien Carbonsäuren verseift werden, aus denen mit entsprechenden Basen erfindungsgemäße Salze erhältlich sind.

5. Amine der allgemeinen Formel (II)

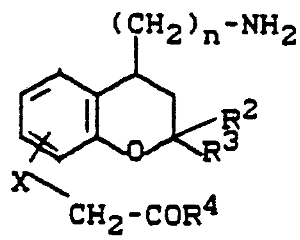

in welcher
$R^2$, $R^3$, $R^4$, X und n die in den Ansprüchen 1 bis 2 angegebene Bedeutung haben.

6. Verwendung von Aminen der allgemeinen Formel (II) gemäß Anspruch 5 zur Herstellung von Chromanen der allgemeinen Formel (I) gemäß Anspruch 1.

7. Arzneimittel, enthaltend mindestens eine Verbindung der allgemeinen Formel (I) gemäß Anspruch 1.

8. Verfahren zur Herstellung von Arzneimitteln, dadurch gekennzeichnet, daß man Verbindungen der allgemeinen Formel (I) gemäß Anspruch 1, gegebenenfalls mit Hilfs- und Trägerstoffen, in eine geeignete Applikationsform überführt.

9. Verwendung von Verbindungen der allgemeinen Formel (I) gemäß Anspruch 1 bei der Bekämpfung von thromboembolischen, ischaemischen, asthmatischen und allergischen Erkrankungen.

10. Verwendung von Verbindungen der allgemeinen Formel (I) gemäß Anspruch 1 bei der Herstellung von Arzneimitteln zur Behandlung von Thrombosen, Thromboembolien, Ischaemien, Asthma und Allergien.